# EUROPÄISCHE PATENTANMELDUNG

(11) **EP 0 885 873 A1**
(43) Veröffentlichungstag der Anmeldung: **23.12.1998**
(21) Anmeldenummer: 98107239.0
(22) Anmeldetag: 21.04.1998
(51) Int. Cl.: C07C 67/08, C07C 69/82

(54) **Methanolyse von Destillationsrückstand der Rohesterdestillation im Witten-Hercules-Verfahren zur Herstellung von Dimethylterephthalat**

(30) Priorität: 10.06.1997 DE 19724390
(71) Anmelder: HÜLS AKTIENGESELLSCHAFT, 45764 Marl (DE)
(72) Erfinder: Neutzler, Ulrich, 58300 Wetter (DE); Schoengen, Anton, 58452 Witten (DE)

(57) **Zusammenfassung**

Die Erfindung betrifft ein Verfahren zur Methanolyse von Destillationsrückstand der Rohesterdestillation im Verfahren zur Herstellung von Dimethylterephthalat nach dem Witten-Hercules-Verfahren, dadurch gekennzeichnet, daß man in die Methanolysezone (3.5) auf mehr als einer Ebene direkt Methanol einführt. Vorteilhaft wird Methanol auf 2 bis 10 Ebenen, insbesondere auf 3 bis 8 Ebenen in die Methanolysezone (3.5) eingeleitet.

## Beschreibung

Die Erfindung betrifft ein verbessertes Verfahren zur Gewinnung von Wertstoffen aus dem Destillationsrückstand der Rohesterdestillation im Witten-Hercules-Verfahren zur Herstellung von Dimethylterephthalat (DMT) unter Abtrennung von unerwünschten Hochsiedern.

### 1. Das Witten-Hercules-Verfahren und die Methanolyse

Nach dem Witten-Hercules-Verfahren wird DMT hergestellt, indem man p-Xylol (PX) in Gegenwart eines Kobalt und Mangan enthaltenden Katalysators in einem ersten Oxidationsschritt zunächst zur p-Toluylsäure (PTS) oxidiert, diese mit Methanol zum p-Toluylsäuremethylester (PTE) verestert, der in einem zweiten Oxidationsschritt katalytisch zum Monomethylterephthalat (MMT) oxidiert wird, das wiederum mit Methanol zu DMT verestert wird. Der erste und der zweite Oxidationsschritt werden dabei ganz oder teilweise im Gemisch der beteiligten Stoffe durchgeführt. In diesem Gemisch wird auch ein Teil der PTS direkt zu Terephthalsäure (TPS) oxidiert, die zusammen mit der übrigen PTS verestert wird und direkt DMT ergibt. Der Oxidationsprozeß, die sogenannte Oxidation, wird mit Sauerstoff oder Sauerstoff enthaltenden Gasen, wie Luft, diskontinuierlich oder kontinuierlich in einzelnen Reaktoren oder mehrstufigen Reaktorkaskaden durchgeführt.

Das bei der Veresterung anfallende, überwiegend DMT und PTE enthaltende Rohestergemisch wird in der sogenannten Rohesterdestillation in einem Kolonnensystem unter Vakuum rektifiziert und in drei Fraktionen aufgeteilt. Die leichtsiedende, den PTE enthaltende Fraktion wird in die Oxidation zurückgeführt. Die mittelsiedende wird DMT-roh genannt, das in weiteren Prozeßstufen, die z.B. aus Destillationen und Kristallisationen bestehen können, weiter gereinigt oder auch zur Herstellung reiner, direkt mit Glykolen veresterbarer Terephthalsäure (TPA) verwendet werden kann. Aus der am höchsten siedenden Fraktion, dem Rückstand (im folgenden als "Destillationsrückstand" bezeichnet). werden in einer Nachbehandlung darin enthaltene Wertstoffe gewonnen. Die Nachbehandlung besteht meistens in einer sogenannten Methanolyse. Aus dem nach der Methanolyse verbleibenden Rückstand (im folgenden als "Methanolyserückstand" bezeichnet) werden üblicherweise die Katalysatorbestandteile mit einer Essigsäure enthaltenden wäßrigen Lösung als Acetate extrahiert und anstatt frischen Katalysators in die Oxidation zurückgeführt. Der Extraktionsrückstand schließlich wird im allgemeinen zur Erzeugung von Energie in einer Rückstandsverbrennungsanlage verbrannt.

Bei der Methanolyse wird der Destillationsrückstand zusammen mit Methanol auf die gewünschte erhöhte Temperatur gebracht, bei der unter Drücken, die üblicherweise um den Normaldruck liegen, die erwünschten Reaktionen ablaufen. Hierbei bilden sich neue Wertstoffe, wie in der Folge näher erläutert wird, die zu den Wertstoffen kommen. die wegen ihrer nur mehr oder weniger vollständigen Abtrennung in der Rohesterdestillation im Destillationsrückstand verblieben sind. Die Wertstoffe destillieren mit dem Methanoldampf ab, werden dephlegmiert und in andere Stufen des DMT-Verfahrens zurückgeführt, wo sie letztendlich in DMT umgewandelt werden. Die mitdestillierenden, im Prozeß aber nicht erwünschten oder gar störenden sogenannten "destillierbaren Hochsieder" werden normalerweise vor der Rückführung der Wertstoffe noch in der Methanolyse destillativ von den Wertstoffen getrennt und gelangen in den Methanolyserückstand.

Die Methanolyse ist ein sehr komplexes Geschehen, bei dem die folgenden Reaktionen und Vorgänge eine Rolle spielen:
(1) Veresterung von noch im Destillationsrückstand verbliebenen nicht veresterten Säuren, wie restliches MMT, TPS und PTS, sowie von Carboxylgruppen. die an Moleküle mit höherem Molekulargewicht gebunden sind, mit Methanol zu den entsprechenden Methylestern:
(2) Umesterung von im Destillationsrückstand enthaltenen hochsiedenden Estern aus aromatischen Alkoholen, wie p-Toluylalkohol (pTA) und p-Hydroxymethylbenzoesäuremethylester, und im Verfahren vorkommenden aromatischen Säuren, wie MMT, TPS und PTS, mit Methanol zu den entsprechenden Methylestern und den dabei freiwerdenden aromatischen Alkoholen;
(3) Veretherung der durch die Umesterung (2) frei werdenden aromatischen Alkohole mit Methanol zu den entsprechenden Methylethern.
   Da in den Reaktionen (1) bis (3) Stoffe entstehen, die relativ leicht flüchtig sind, d.h. unter den Bedingungen der Methanolyse destillieren oder durch den heißen Methanoldampf gestrippt werden, wird das Gleichgewicht der Veresterungs- und Umesterungsreaktionen ständig in erwünschter Weise in Richtung auf die Entstehung der gewünschten leichter siedenden Wertstoffe verschoben. Daneben werden auch die im Destillationsrückstand ursprünglich vorhandenen relativ leicht flüchtigen Wertstoffe ohne chemische Veränderung abdestilliert bzw. durch den heißen Methanoldampf abgestrippt. Zu diesen Wertstoffen zählen:
(4) DMT sowie seine ebenfalls relativ leicht flüchtigen Vorläuferstoffe, wie PTE, Terephthalaldehydsäuremethylester (TAE). HMBME, p-Methoxymethylbenzoesäuremethylester (MMBME) und ähnliche Stoffe;.
   Neben diesen erwünschten Umsetzungen und Trennoperationen gibt es eine ausgesprochen unerwünschte, nämlich
(5) das Abstrippen relativ leicht flüchtiger. aber höher als die Wertstoffe siedende Stoffe, wie Trimellithsäuretrimethylester (TMT), relativ leicht flüchtige Diphenyle und ähnliche Rückstandsbestandteile ("destillierbare Hochsieder"), die bei der Rückführung in andere Stufen des DMT-Verfahrens stören und daher von den Wertstoffen abgetrennt werden müssen.
   Weiterhin gibt es eine Reihe von weiteren, überwiegend durch hohe Temperaturen begünstigte unerwünschte Nebenreaktionen, nämlich
(6) Rückreaktionen der beschriebenen Veresterungs- und Umesterungsreaktionen, die stets zu Gleichgewichten führen; diese Rückreaktionen laufen namentlich in schlecht durchmischten oder rückvermischten Zonen der Apparatur ab;
(7) Kondensationsreaktionen, die - wohl unter dem Einfluß der katalytisch wirkenden Kobaltbestandteile des Destillationsrückstands - ebenfalls vorwiegend in schlecht durchmischten, hochtemperierten Zonen der Apparatur ablaufen und zur Bildung mehrkerniger, hochzäher Produkte führen;
(8) thermolytische Spaltprozesse (Crackprozesse) an heißen Oberflächen und in überhitzten Anteilen des Methanolysegemisches, bei denen Kohlendioxid und vorwiegend nicht in DMT umwandelbare und daher nicht erwünschte leichtsiedende Stoffe entstehen;
(9) Bildung schlecht löslicher, z.T. koksartiger Kobaltverbindungen aus Stoffen, die durch die Crack- und Umesterungsprozesse entstanden sind, in hochtemperierten Zonen;
(10) Abscheidung von metallischem Kobalt durch Reduktion von Kobaltverbindungen durch Methanol bzw. darin enthaltene reduzierende Stoffe, wie Formaldehyd. namentlich an heißen Wänden.
   Während die Rückreaktionen (6) hauptsächlich die Produktivität nachteilig beeinflussen , sind die Kondensations- und Crackprozesse gemäß (7) und (8) in erster Linie ausbeutemindernd. Die Reaktionen gemäß (9) und (10) beeinflussen die Kobaltbilanz sehr ungünstig, da die abgelagerten kobalthaltigen Stoffe sich der nachfolgenden Extraktion entziehen und der so entstehende scheinbare Katalysatorverlust durch Frischkatalysator ersetzt werden muß. Außerdem lagern sich die gebildeten schwerlöslichen Stoffe in Rohrleitungen und Apparateteilen ab und verursachen dadurch häufige Betriebsunterbrechungen und erhöhten Reinigungsaufwand. Die gemäß (7) gebildeten Stoffe verbleiben zu einem großen Teil im Methanolyserückstand und beeinflussen deshalb die nachfolgende Extraktion nachteilig, weil es nur bei dünnflüssiger Konsistenz des Rückstands zu einer weitgehenden Rückgewinnung der Katalysatormetalle kommt. Ist die Zähigkeit des Methanolyserückstands zu groß, müssen vor der Extraktion größere Mengen an verdünnenden Hilfslösemitteln zugesetzt werden, die nach der Extraktion wieder kostenaufwendig abgetrennt werden müssen. Da die Rückstande nach der Extraktion normalerweise verbrannt werden, stört auch hier eine zu hohe Zähigkeit erheblich, da hochzähe Stoffe sich kaum pumpen oder anderweitig transportieren und eindüsen lassen.

### 2. Anlagen zur Methanolyse nach dem Stand der Technik

### 2.1 Frühe einstufige Anlagen

In Anlehnung an den verfahrenstechnisch ähnlichen Veresterungsprozeß wurden ursprünglich als Methanolysereaktoren einfache kolonnenartige, außenbeheizte Reaktoren mit eingebauten Reaktionsböden und aufgesetztem Destillationsteil zur Abtrennung der erwähnten destillierbaren Hochsieder benutzt. Diese Reaktoren enthielten im Sumpfteil einen Verteiler zur Aufgabe des dampfförmigen Methanols. Die hohen Temperaturen an den Reaktorwänden begünstigten jedoch die unter (6) bis (10) aufgeführten Nebenreaktionen. Zudem zeigte sich, daß die heißen Wände der Reaktoren leicht verkrusteten und die abblätternden Feststoffe zusammen mit anderen Feststoffen, die im Destillationsrückstand vorhanden waren oder sich bildeten, die Reaktionsböden verstopften, wodurch häufige Betriebsunterbrechungen mit Reinigungsarbeiten erforderlich wurden.

### 2.2 Verbesserte einstufige Anlagen

Eine Verbesserung dieser ursprünglichen Methanolysen mit außenbeheiztem Reaktor waren Systeme mit Reaktoren ohne Reaktionsböden, in denen neben der Methanoleinleitung im Sumpf ein Umlauf von Methanolysegemisch mit einem außenliegenden Umlaufwärmetauscher vorhanden war, wobei in den Umlauf gegebenenfalls weiteres Methanol eingeleitet wurde. In diesen Systemen gelingt es, bei hinreichend großem Produktumlauf die für die Methanolysereaktionen und die Strippung der Wertstoffe erforderliche Wärme ohne Außenheizung des Reaktors schonend einzubringen. Dadurch wurden die unter (6) und (10) aufgeführten störenden Nebenreaktionen deutlich zurückgedrängt.

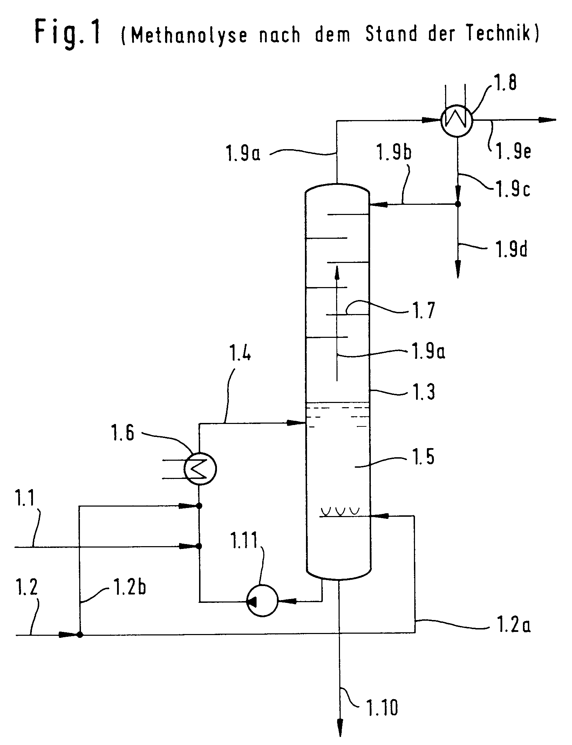

Eine solche Anlage zur Durchführung einer Methanolyse ist in Figur 1 dargestellt. Der Destillationsrückstand 1.1 wird unterhalb des Umlaufwärmetauschers 1.6 in den Umlauf 1.4 eingeführt und mit umlaufendem Methanolysegemisch in den oberen Teil der Reaktionszone 1.5 des Reaktors 1.3 eingeführt. Das Methanol wird in zwei Teilströme 1.2a, im folgenden "Reaktionsmethanol" genannt, und 1.2b, im folgenden "Strippmethanol" genannt, aufgeteilt. Natürlich bewirkt das Methanol beider Teilströme sowohl eine Methanolyse des Destillationsrückstands, als auch das Abstrippen flüchtiger Stoffe. Die gewählten Bezeichnungen sollen lediglich Wirkungsschwerpunkte kennzeichnen. Das Reaktionsmethanol 1.2a wird unmittelbar in den Reaktor 1.3 eingeführt. das Strippmethanol 1.2b mittelbar über den Umlauf 1.4.

In der Methanolysezone 1.5, die praktisch das Flüssigkeitsvolumen im Reaktor 1.3 umfaßt, findet in der vom Methanoldampf 1.2a durchströmten Flüssigkeit die Methanolyse bei Temperaturen von 180°C bis 300°C unter Atmosphärendruck oder leicht vermindertem oder erhöhtem Druck statt. Die hierfür und für das Strippen der flüchtigen Wertstoffe erforderliche Wärme wird durch den z.B. mit einem Wärmeträgeröl beheizten Umlaufwärmetauscher 1.6 über den Umlauf 1.4 zugeführt, unterhalb dessen das Strippmethanol 1.2b eingeführt wird.

Die unter (1) bis (4) aufgeführten Wertstoffe sowie die destillierbaren Hochsieder gemäß (5) bilden mit Methanoldampf den Brüdenstrom 1.9a, der aus der Methanolysezone 1.5 in den Destillationsteil 1.7, eine aufgesetzte Kolonne ohne Abtriebsteil, eintritt. Die destillierbaren Hochsieder gemäß (5) beeinflussen die Oxidation sehr nachteilig und müssen daher vor der Rückführung der Wertstoffe abgetrennt werden. Hierzu wird der Brüdenstrom 1.9a im Destillationsteil 1.7 mittels Rücklauf 1.9b, der ein Teil des im Dephlegmator 1.8 ausgekühlten Dephlegmats 1.9c ist, einer rektifizierenden Trennoperation unterworfen. Dabei gelangen die destillierbaren Hochsieder als Bestandteil des Flüssigkeitsrücklaufs aus dem Destillationsteil 1.7 wieder in die Methanolysezone 1.5 zurück und werden schließlich mit dem Methanolyserückstand 1.10 abgezogen, aus dem, wie beschrieben, die Katalysatorbestandteile extrahiert werden.

Die Pumpe 1.11 bewirkt den Umlauf 1.4 der Flüssigkeit, die dem Sumpf des Reaktors 1.3 entnommen wird, Die Pumpe kann entfallen, wenn dem Umlauf 1.4 genügend Strippmethanol zugeführt und dadurch die nötige Volumenverdrängung bewirkt wird.

Die Wertstoffe werden entweder als flüssige Wertstoffe 1.9d oder zusammen mit dampfförmigem Methanol im Restbrüden 1.9e in andere Stufen des DMT-Verfahrens zurückgeführt.

### 2.3 Zweistufige Methanolyse

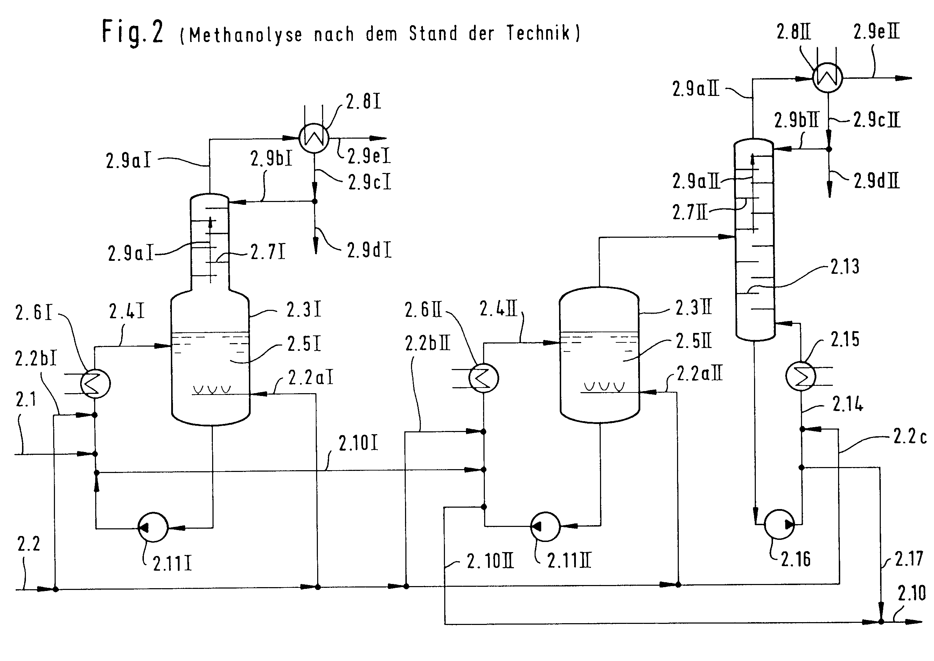

Die Abtrennung der destillierbaren Hochsieder in der aufgesetzten Kolonne des Destillationsteils 1.7 gelingt allerdings auf befriedigende Weise nur dann, wenn der Destillationsrückstand nicht zu große Mengen an Wertstoffen, insbesondere an DMT, enthält. Anderenfalls muß die Temperatur in der Methanolysezone erheblich gesteigert werden, um die für eine Abtrennung der destillierbaren Hochsieder erforderlichen hohen Rücklaufmengen zu erzeugen. Dies fördert einerseits die unerwünschten Nebenreaktionen gemäß (7) bis (10). Andererseits belasten die für das Strippen erforderlichen hohen Methanolmengen das System und können in nachfolgenden Verfahrensstufen nicht mehr oder nicht mehr ohne weiteres verarbeitet werden. In solchen Fällen kann die in DE-A1 139 05 586 beschriebene und in der Figur 2 dargestellte zweistufige Methanolyse zur Abtrennung der destillierbaren Hochsieder dienen. In der ersten Stufe im linken Teil der Figur entspricht die Darstellung (und entsprechen die Bezeichnungen) der Figur 1. In der Methanolysezone 2.5 I dieser Stufe herrschen in der Regel Temperaturen von 180°C bis 300°C. Der aus dem Umlauf 2.4 I entnommene Rückstand 2.10 I der ersten Stufe wird in die zweite Stufe im rechten Teil der Figur überführt, die wiederum weitgehend der Figur 1 entspricht. jedoch mit einem getrennten Destillationsteil 2.7 II mit einem Abtriebsteil 2.13, einem Umlauf 2.14 mit Umlaufwärmetauscher 2.15 und Pumpe 2.16 sowie, zur Absenkung der Sumpftemperatur, einer eigenen Einleitung für Strippmethanol 2.2c versehen ist. In der Methanolysezone 2.5 II der zweiten Stufe herrscht im allgemeinen eine Temperatur von 180°C bis 300°C. Der Brüdenstrom 2.9a II wird, ähnlich wie gemäß Figur 1. in das Dephlegmat 2.9c II, das wiederum in Rücklauf 2.9b II und sowie flüssige Wertstoffe 2.9d II aufgeteilt wird, sowie in den Restbrüden 2.9e II aufgetrennt.

Bei der zweistufigen Methanolyse wird also der Methanolyserückstand 2.10 I der ersten Stufe einer erneuten Methanolyse in der zweiten Stufe unterworfen. Die destillierbaren Hochsieder 2.17 gelangen aus dem Sumpf des Abtriebsteils 2.13 in den Rückstand 2.10 II und werden mit diesem als Methanolyserückstand 2.10 ausgeschleust.

### 3. Aufgabe der Erfindung

Die zweistufige Methanolyse nach DE-A1 39 04 586 arbeitet zwar schonender und mit höherer Rückgewinnung von Wertstoffen als die zuvor beschriebenen Methanolysen älterer Bauart, ist aber viel aufwendiger. Dies gilt nicht nur für die Investitionskosten, sondern auch auch für die Betriebs- und Wartungskosten. Es wäre daher erwünscht, ein Verfahren für die Methanolyse des Destillationsrückstands zu haben, das mindestens so schonend und effizient arbeitet. aber hinsichtlich der Investitions-, Betriebs- und Wartungskosten günstiger ist als die zweistufige Methanolyse.

### 4. Kurzbeschreibung und Vorteile der Erfindung

Es wurde überraschend gefunden, daß sich die Methanolyse von Destillationsrückstand der Rohesterdestillation in der Herstellung von DMT nach dem Witten-Hercules-Verfahren in diesem Sinne erheblich verbessern läßt. wenn man in die Methanolysezone auf mehr als einer Ebene direkt Methanol einleitet. Das Methanol kann in flüssiger Form eingeleitet werden, vorteilhaft wird es jedoch zumindest teilweise dampfförmig angewandt.

Es ist empfehlenswert, mindestens einen Umlauf mit Umlaufwärmetauscher und zweckmäßig einer Pumpe vorzusehen, weil so die problematische Wandbeheizung der Reaktoren entfallen kann. Bei hinreichend hohem Produktumlauf wird die für die Methanolysereaktionen und das Abstrippen der Wertstoffe erforderliche Wärme schonend zugeführt. Die Temperatur des Wärmeträgermittels braucht dann nur wenig, z.B. um bis zu etwa 30°C, über der Methanolysetemperatur zu liegen. Es ist weiterhin empfehlenswert, diesen Umlauf zugleich als indirekte Methanoleinleitung zu benutzen. Ein zweiter und gegebenenfalls sogar ein dritter Umlauf mit indirekter Methanoleinleitung in die Methanolysezone sowie mit einer zugeordneten direkten Methanoleinleitung ist bzw. sind besonders dann zweckmäßig, wenn der Destillationsrückstand - aus anlagespezifischen Gründen oder infolge von Störungen - größere Mengen an DMT und/oder hochmolekularen Stoffen enthält.

Es ist ein wesentliches Merkmal des Verfahrens nach der Erfindung, daß in die Methanolysezone auf mehr als einer Ebene direkt Methanol eingeleitet wird. Vorteilhaft leitet man auf 2 bis 10, insbesondere auf 3 bis 8 Ebenen Methanol ein. Es ist jedoch empfehlenswert, mindestens eine indirekte Einleitung über einen Produktumlauf vorzusehen.

Im Rahmen der Untersuchungen, die zu der vorliegenden Erfindung führten, wurde gefunden, daß die Reaktionsgeschwindigkeiten der Methanolysereaktionen im Gegensatz zu der bisher herrschenden Auffassung verhältnismäßig hoch und die Verweilzeiten in der Methanolysezone daher vergleichsweise gering sind. Die Rückreaktionen werden durch die erfindungsgemäße Verfahrensweise, die durch das Gegenstromprinzip, stufenweise Methanolaufgabe und intensive Strippung gekennzeichnet ist, minimiert. Das Verfahren nach der Erfindung erlaubt es daher, in einem gegebenen Apparatevolumen eine gegebene Menge Destillationsrückstand bei im Vergleich zu konventionellen Methanolysen niedrigerer Temperatur aufzuarbeiten und den Destillationsrückstand ohne nennenswerte Abscheidungen oder gar Verstopfungen optimal zu verwerten. Alternativ gelingt es, bei einem gegebenen Apparatevolumen bei einer im Vergleich zu konventionellen Methanolysen gleichen Temperatur die Raum-Zeit-Ausbeute wesentlich zu verbessern. Im ersteren Fall wird die DMT-Ausbeute des Gesamtverfahrens um mehrere Prozent verbessert, da infolge der niedrigeren Temperatur die unerwünschten Reaktionen (6) bis (10) weitergehend zurückgedrängt werden. Wegen der niedrigeren Temperaturen können auch die destillierbaren Hochsieder bei verminderten Rückläufen selbst durch einfache aufgesetzte Destillationskolonnen im Methanolyserückstand gehalten werden. Der Methanolyserückstand ist bei den Extraktionstemperaturen so dünnflüssig. daß die Katalysatorextraktion unproblematisch ist.

Bei Neuanlagen ergeben sich durch die einfache Form der kolonnenartigen Reaktoren mit aufgesetztem Destillationsteil, die sich für die Durchführung des Verfahrens besonders eignen, erhebliche Investitionsvorteile. Das erfindungsgemäße Verfahren läßt sich aber auch in allen bestehenden Anlagen ohne größere Änderungen durchführen. Besonders große Verbesserungen ergeben sich bei schlanken, kolonnenartigen Methanolysereaktoren. wenn die Zahl der Einleitebenen auf 4 bis 5 erhöht werden kann. Bei weniger schlanken. vor allem in jüngeren Anlagen vorkommenden Methanolysereaktoren sind jedoch meistens nur 1 oder 2 zusätzliche direkte Einleitebenen sinnvoll, da sich die Einleitungen sonst gegenseitig nachteilig beeinflussen. Das Verfahren nach der Erfindung läßt sich auch bei zwei- und gegebenenfalls mehrstufigen Methanolysen anwenden. Dann bleibt es zwar bei den vergleichsweise hohen Investitions-, Betriebs- und Wartungskosten, es werden jedoch auch bei Belastung mit erheblichen Mengen an Destillationsrückstand mit relativ hohen Wertstoffkonzentrationen größere Anteile dieser Wertstoffe als bisher zurückgewonnen.

### 5. Ausführliche Beschreibung der Erfindung

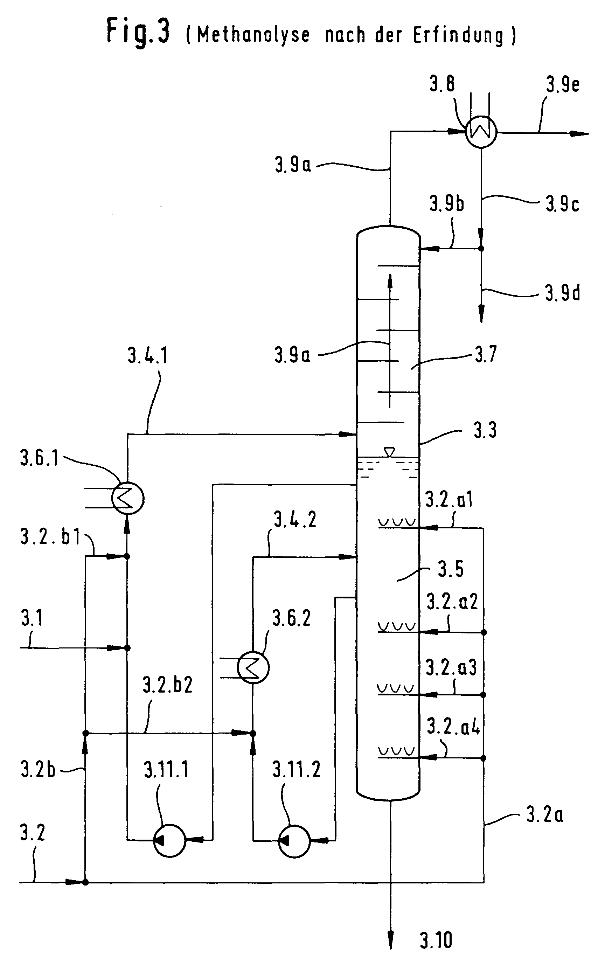

Eine Anlage zur Durchführung des Verfahrens nach der Erfindung ist in Figur 3 dargestellt. Sie ähnelt der Anlage nach der Figur 1, und die Bezeichnungen entsprechen daher einander. Der Reaktor 3.3 hat vier direkte Methanoleinleitungen 3.2a.1 bis a.4 sowie zwei Umläufe von Methanolysegemisch 3.4.1 und 3.4.2 mit indirekten Methanoleinleitungen 3.2.b1 und 3.2.b2. Umlaufwärmetauschern 3.6.1 und 3.6.2 sowie Pumpen 3.11.1 und 3.11.2. Die Pumpen können ganz oder zum Teil entfallen, wenn durch Einleitung genügender Mengen von gegebenenfalls vorerhitztem Methanol in den jeweiligen Umlauf eine hinreichende Verdrängungswirkung erzielt wird. Der obere Produktumlauf enthält die Einspeisung für den Destillationsrückstand 3.1. der mit dem eingeleiteten Methanol 3.2.b1 und dem umlaufenden Methanolysegemisch auf den Brüdenraum unterhalb der aufgesetzten Kolonne 3.7 geführt wird, der als Flüssigkeitsabscheider fungiert. Die abgetrennten Dämpfe vereinigen sich mit den aus der Methanolysezone 3.5 aufsteigenden Dämpfen zum Brüden 3.9a, und dieser wird in der Kolonne 3.7 den beschriebenen Trennoperationen zur Abscheidung der destillierbaren Hochsieder unterworfen. Diese werden in der aus der Kolonne 3.7 ablaufenden Flüssigkeit angereichert und letztlich mit dem Methanolyserückstand 3.10 ausgetragen. Das Methanolysegemisch für den oberen Umlauf 3.4.1 wird oberhalb der obersten direkten Methanoleinleitung entnommen. Das Methanolysegemisch für den unteren Umlauf 3.4.2 wird oberhalb der nächst tieferen direkten Methanoleinleitung 3.2.a2 entnommen, mit indirektem Methanol 3.2.b2 vermischt, durch den Umlaufwärmetauscher 3.6.2 geführt und in demselben Bereich der Methanolysezone 3.5 oberhalb der direkten Methanoleinleitung 3. 2.a2 wieder aufgegeben. Unterhalb dieser zweiten direkten Methanoleinleitung sind noch zwei weitere 3.2.a3 und 3. 2.a4 im Sumpfteil der Methanolysezone 3.5 vorhanden.

Die am Kopf der aufgesetzten Kolonne 3.7 austretenden, mit flüchtigen Wertstoffen beladenen Brüden 3.9a werden im Dephlegmator 3.8 so weit abgekühlt, daß sich Dephlegmat 3.9c abscheidet, das zum Teil als Rücklauf 3.9b in die Kolonne 3.7 und zum Teil als flüssige Wertstoffe 3.9d in andere Stufen des DMT-Verfahrens zurückgeführt wird. Der nicht kondensierte Restbrüden 3.9e, der weiterhin Wertstoffe enthält, wird ebenfalls in andere Stufen des DMT-Verfahrens zurückgeführt.

Es wird dem Fachmann deutlich sein, daß die angegebene Zahl der direkten und indirekten Methanoleinleitungen sowie deren Anordnung gemäß Figur 3 nur beispielhaft und andere erfindungsgemäße Ausführungsformen möglich sind, die alle dadurch gekennzeichnet sind, daß in die Methanolysezone auf mehr als einer Ebene direkt und gegebenenfalls zusätzlich indirekt Methanol eingeleitet wird.

Es ist möglich, die in die Umläufe eingegebenen Stoffströme vermischt oder unvermischt vorzuwärmen, um die Temperaturdifferenz im Umlaufwärmetauscher möglichst gering halten und dadurch den Destillationsrückstand schonend erhitzen zu können. Um die thermische Belastung des Destillationsrückstands zu vermindern, kann man ihn auch, zweckmäßig mit indirektem Methanol, auf mehrere Umläufe verteilen.

Der Destillationsrückstand, der in dem Verfahren nach der Erfindung der Methanolyse unterworfen wird, fällt bei der Destillation des Reaktionsgemisches der Veresterungsstufe im DMT-Verfahren an und enthält neben den nicht destillierbaren Katalysatorbestandteilen und hochmolekularen, kaum destillierbaren Stoffe, je nach Anlage und deren Betriebsweise, noch 2% bis 30% DMT sowie noch nicht veresterte Säuren. Ester aromatischer Alkohole mit den in der Oxidation erzeugten Säuren sowie die anderen unter (1) bis (5) angesprochenen Stoffe. In der Methanolyse entstehen aus diesen Stoffen die unter (1) bis (3) erwähnten Reaktionsprodukte, die zusammen mit den unter (4) und (5) angegebenen im Brüden 3.9a in die aufgesetzte Kolonne 3.7 gelangen und dort wegen der niedrigen Temperaturen im erfindungsgemäßen Verfahren mit geringen Rückläufen 3.9b von den ebenfalls mitdestillierenden unerwünschten Hochsiedern gemäß (5) getrennt werden und als flüssige Wertstoffe 3.9d bzw. im Restbrüden 3.9e in vorhandene Stufen des DMT-Verfahrens zurückgeführt werden.

Im allgemeinen wendet man das Methanol, aufgeteilt auf die verschiedenen Einleitungen, in der 0.5- bis 10-fachen Menge des Destillationsrückstands an. Dabei sollte ein erheblicher Teil, z.B. 25% bis 60%, der Gesamtmenge indirekt über den entsprechend ausgelegten oberen oder obersten Produktumlauf und ein kleinerer Teil, z.B. 15% bis 25%, auf die ihm zugeordnete direkte Einleitung entfallen. Das restliche Methanol wird auf die weiteren direkten und indirekten Einleitungen verteilt, wobei es günstig ist, wenn 30% bis 60% dieser Restmenge auf den nächst tieferen Produktkreislauf, falls vorhanden, und die ihm zugeordnete direkte Einleitung gegeben und das dann noch verbleibende Methanol auf die übrigen direkten und bzw. oder indirekten Einleitungen verteilt wird. Die optimale Verteilung des restlichen Methanols hängt u.a. von den geometrischen Verhältnissen des Reaktors ab und kann durch Variation der Mengen ermittelt werden.

Unabhängig von der Zahl und der Art der Methanoleinleitungen - direkt oder indirekt- herrscht in der Methanolysezone zweckmäßig eine Temperatur von 200°C bis 275°C, vorteilhaft von 220°C bis 260°C. Die Temperatur wird, wie zuvor erwähnt, vorteilhaft durch Umlauf von Methanolysegemisch über einen oder mehrere außenliegende Umlaufwärmetauscher aufrechterhalten. Man arbeitet, wie bei den bekannten Verfahren, zweckmäßig bei Atmosphärendruck oder unter leicht erhöhtem oder vermindertem Druck von 0.8 bis 3.0 bar, vorzugsweise bei 1.0 bis 2.0 bar, am Kopf des Reaktors. Höhere Drücke sind zwar möglich, aber unwirtschaftlich, da sie die Rückreaktion gemäß (6) fördern.

Bei dem Verfahren nach der Erfindung wird der Destillationsrückstand in einen Methanolyserückstand 3.10, der noch 0,5 bis 2,5 %, vorzugsweise etwa 1% DMT sowie die destillierbaren Hochsieder, alle nicht destillierbaren hochmolekularen organischen Rückstände sowie die Katalysatorbestandteile enthält. und in eine flüchtige. das Methanol enthaltende Fraktion getrennt, aus der ein auch als Rücklauf 3.9b verwendetes Dephlegmat 3.9c abgeschieden wird. Es ist unzweckmäßig, die Methanolyse so zu gestalten, daß auch die im Methanolyserückstand 3.10 noch enthaltenen geringen Mengen an DMT destilliert oder gestrippt werden. Dies wäre zwar im Interesse einer hohen DMT-Ausbeute erwünscht, ließe aber den Methanolyserückstand 3.10 unnötig zäh werden, so daß er, wie erwähnt, schwierig zu extrahieren und zu verbrennen wäre.

Für das Verfahren nach der Erfindung eignen sich im Prinzip alle Reaktoren, die, von den Methanoleinleitungen abgesehen, den für übliche Methanolyseverfahren verwendeten Reaktoren entsprechen. Sie haben also in der Regel einen Durchmesser D von 0,5m bis 5m oder mehr, sind zylindrisch und von 2 bis 10 x D lang, haben im unteren, ca. 50% oder mehr des Volumens umfassenden Teil, der Methanolysezone, bis auf die Methanoleinleitungen keine Einbauten, haben im verbleibenden oberen Teil vorzugsweise Einrichtungen zur Flüssigkeits/Dampf-Trennung und, je nach Bauart, eine aufgesetzte Kolonne ohne Abtriebsteil, eine getrennte Kolonne mit oder ohne Abtriebsteil oder eingebaute Trennelemente üblicher Bauart zur Verstärkung der destillativen Trennwirkung.

Die mittleren Leerrohrgeschwindigkeiten in der Methanolysezone des Reaktors sollten in der Regel 15 cm/sec, vorteilhaft 10 cm/sec nicht wesentlich überschreiten, damit die Strömungsverhältnisse nicht zu ungünstig werden. Der Abstand der Ebenen, in denen dem Reaktor direkt oder indirekt Methanol zugeführt wird, sollte mindestens 0,3 und bis zu 3 x D und vorzugsweise 1 bis 2 x D betragen. Bei indirekter Zuführung wird der Abstand zwischen der Stellen gemessen, an der das umlaufende, mit Methanol versetzte Methanolysegemisch wieder in den Reaktor eintritt, und der nächsten Stelle, an der direktes oder indirektes Methanol eingeleitet wird. Auf jeden Fall sollten Umläufe sich nicht überschneiden (d.h. zwischen Entnahme- und Zuführungsstelle eines Kreislaufs sollte sich keine Entnahme- und/oder Zuführungsstelle eines anderen Umlaufs befinden).

## Patentansprüche

1. Verfahren zur Methanolyse von Destillationsrückstand der Rohesterdestillation im Verfahren zur Herstellung von Dimethylterephthalat nach dem Witten-Hercules-Verfahren, dadurch gekennzeichnet, daß man in die Methanolysezone (3.5) auf mehr als einer Ebene direkt Methanol einführt.

2. Verfahren nach Anspruch 1, dadurch gekennzeichnet, daß man Methanol auf 2 bis 10 Ebenen in die Methanolysezone (3.5) einleitet.

3. Verfahren nach Anspruch 1, dadurch gekennzeichnet, daß man Methanol auf 3 bis 8 Ebenen in die Methanolysezone (3.5) einleitet.

4. Verfahren nach einem der Ansprüche 1 bis 3, dadurch gekennzeichnet, daß mindestens ein Umlauf (3.4.1, 3.4.2) von Methanolysegemisch mit außenliegendem Umlaufwärmetauscher (3.6.1, 3.6.2) vorhanden ist. über den die für die Methanolysereaktionen und für das Abstrippen der Wertprodukte erforderliche Wärme ganz oder teilweise eingebracht wird.

5. Verfahren nach Anspruch 4, dadurch gekennzeichnet. daß der Umlauf bzw. die Umläufe zugleich als indirekte Methanoleinleitung(en) benutzt wird bzw. werden.

6. Verfahren nach Anspruch 4, dadurch gekennzeichnet, daß das Methanolysegemisch des oberen oder obersten Umlaufs (3.4.1) oberhalb des Flüssigkeitsspiegels des Methanolysegemisches in den Methanolysereaktor 3.3 zurückgeführt wird.

7. Verfahren nach einem der Ansprüche 1 bis 6, dadurch gekennzeichnet, daß der Destillationsrückstand (3.1) ganz oder teilweise über einen oder mehrere Umläufe (3.4.1, 3.4.2) in die Methanolysezone (3.5) eingeführt wird.

8. Verfahren nach einem der Ansprüche 1 bis 7, dadurch gekennzeichnet, daß die in die Methanolysezone (3.5) eingeführten Ströme vorher für sich allein oder gemischt erwärmt werden,

9. Verfahren nach einem der Ansprüche 1 bis 8, dadurch gekennzeichnet, daß die Temperatur in der Methanolysezone 3.5 200°C bis 275°C beträgt.

10. Verfahren nach einem der Ansprüche 1 bis 8, dadurch gekennzeichnet, daß die Temperatur in der Methanolysezone 3.5 220°C bis 260°C beträgt.

11. Verfahren nach einem der Ansprüche 1 bis 10, dadurch gekennzeichnet, daß die mit Wertstoffen und destilierbaren Hochsiedern beladenen ßrüden 3.9a in eine aufgesetzte Kolonne ohne Abtriebsteil geleitet werden, in der die Wertstoffe von den destillierbaren Hochsiedern getrennt werden, die in den Methanolyserückstand 3.10 gelangen.

12. Verfahren nach einem der Ansprüche 1 bis 11, dadurch gekennzeichnet, daß der Abstand der Ebenen, auf denen Methanol direkt oder indirekt eingeleitet wird, mindestens 0,3 und bis zu 3 x D beträgt, wobei D den Durchmesser des Methanolysereaktors bezeichnet.

13. Verfahren nach einem der Ansprüche 1 bis 12, dadurch gekennzeichnet, daß das Methanol wenigstens teilweise dampfförmig angewandt wird.
